# EUROPEAN PATENT SPECIFICATION

(11) **EP 3 149 488 B1**
(45) Date of publication and mention of the grant of the patent: **17.10.2018**
(21) Application number: 15732042.5
(22) Date of filing: 26.03.2015
(51) Int. Cl.: G01N 33/68

(54) **METHOD FOR DIAGNOSIS OF COLORECTAL CANCER BY DETERMINING THE LEVEL OF FATTY ACIDS PRESENT IN A BIOLOGICAL FLUID SAMPLE, AND APPARATUS FOR IMPLEMENTING THE METHOD**
VERFAHREN UND VORRICHTUNG ZUR DIAGNOSE VON KOLOREKTALKREBS MITTELS BESTIMMUNG DES FETTSÄURESPIEGELS IN BIOLOGISCHEN FLÜSSIGKEITSPROBEN.
MÉTHODE POUR LE DIAGNOSTIC DE CANCER COLORECTAL PAR LA DÉTERMINATION DU TAUX D'ACIDES GRAS PRÉSENTS DANS UN ÉCHANTILLON DE LIQUIDE BIOLOGIQUE, ET APPAREIL POUR LA MISE EN UVRE DE LADITE MÉTHODE

(30) Priority: 28.03.2014 IT VE20140019
(43) Date of publication of application: 05.04.2017
(73) Proprietor: DANI Instruments SA, 6594 Contone (CH)
(72) Inventor: SAINI FASANOTTI, Massimiliano, 6594 Contone (CH); BERGNA, Manuela, 20052 Monza (IT); CROTTI, Sara, 35127 Padova (IT); AGOSTINI, Marco, 35035 Mestrino (Padova) (IT)
(74) Representative: Piovesana, Paolo
(86) International application number: PCT/IB2015/052232
(87) International publication number: WO 2015/145384

(56) References cited:
- WO-A1-2009/098354
- WO-A2-2012/058559
- AU-A1- 2007 202 944
- US-A1- 2012 040 383

## Description

The present invention relates to a method for the diagnosis of colorectal cancer by determining the level of fatty acids present in a biological fluid sample, and an apparatus for implementing the method.

The development of pathological states at the systemic level is known to cause a modification in the metabolic profile in both plasma and urine. This aspect is at the basis of clinical diagnostic methodologies: for example to diagnose diabetes the glycemic level is measured, to diagnose prostate tumour the PCA3 level is measured, etc... Because of this, the development of new high specificity methods and their possible clinical laboratory use for controlling biochemical parameters related to specific pathologies is of considerable interest.

In the case of development of certain types of cancer, metabolic changes have also been observed, resulting in significant analytical alterations in the plasmatic profile of saturated organic acids.

At present, the analytical method for obtaining data of diagnostic interest on organic acids involves the extraction of the organic acids from the plasma, their derivatization, and finally analysis by gas chromatography-mass spectrometry (GC-MS) systems operating under traditional chromatography conditions. In greater detail, these conditions generally involve the use of chromatography columns of 30-60 metres in length, temperature gradients from 40 to 300°C, mass spectrometry operating under electronic ionization conditions, and quadrupole mass analyzers. At present, the steps of said analytical method are carried out separately and are substantially discontinuous, this deriving mainly from the fact that the equipment required to perform the various steps is usually located in different analysis laboratories, or at least in different zones substantially spaced apart.

In this respect, US 2012/040383 describes a metabolite evaluation method for the diagnosis, the monitoring and the treatment of colorectal cancer. This method is not completely satisfactory in that:
- it presents a large number of metabolites (about 65) as biomarker, some of which are influenced by various factors unrelated to the specific pathological state, (for example diet, taking of drugs, lifestyle or other pathological states): in greater detail, the plurality of metabolites used includes long-chain fatty acids (C16-C20), but not medium chain fatty acids (C6-C12),
- for quantitative analysis of metabolites, molecule analogues of the metabolites of interest are used as internal standards, but not the same metabolites labelled with stable isotopes,
- the validity of the identification of colorectal cancer obtained by these metabolites is demonstrated on the basis of overall statistical data, in particularly by analysis of the main components (P.C.A), and in this respect no threshold value is provided which would characterise the pathological state, neither is any R.O.C. curve of the individual metabolites reported for evaluating the validity of the method as a clinically usable diagnostic test,
- it presents no comparison with metabolic profiles of other neoplasms, hence the sensitivity of the method as a diagnostic test for colorectal cancer was not demonstrated.

WO 2009/098354 describes a method of diagnosing irritable bowel syndrome (IBS), and not colorectal cancer. Moreover, this method is particularly invasive given that the analysis is carried out on tissue obtained by biopsy.

WO 2012/058559 and AU 2007 202944 generally describe particularly complex and costly systems for automating the movement and preparation of samples, and their analysis.

The main object of the invention is to propose a simple and reliable method for fatty acid determination of high diagnostic value for colorectal cancer, where "high diagnostic value" means a value substantially exceeding 0.7 on the R.O.C curve (receiver operating characteristic) in biological fluid samples. This parameter is defined with reference to the Swets scale (Swets JA, "Measuring the accuracy of diagnostic systems." in "Science" 3 June 1988; volume 240, no 4857, page. 1285-1293) shown hereinafter, in which A.U.C. ("*Area Under Curve*") indicates the area below the R.O.C. curve. In particular:
- if A.U.C = 0.5 the test is not significant,
- if 0.5 < A.U.C ≤ 0.7 the test is of low accuracy,
- if 0.7 < A.U.C ≤ 0.9 the test is moderately accurate,
- if 0.9 < A.U.C ≤ 1.0 the test is highly accurate,
- if A.U.C = 1.0 the test is perfect.

Another object of the invention is to propose a method which is completely automated and does not require direct manipulation of the biological fluid samples, and can hence guarantee on the one hand a greater safety for the operator, and on the other hand a higher reproducibility.

Another object of the invention is to propose a method which has high specificity (i.e. able to correctly identify healthy subjects) and high sensitivity (i.e. able to correctly identify unwell subjects) in diagnosing the colorectal cancer pathological state, by reducing the number of false positives.

Another object of the invention is to propose a method which enables early diagnosis of colorectal cancer and which, in particular, is able to distinguish between subjects at risk (for example with ulcerative rectocolitis) and unwell subjects.

Another object of the invention is to propose a method which has high organ specificity and which provides values correlatable with the stage of advancement in pathology, with prognosis, and with clinical variations.

Another object of the invention is to propose a method which enables analysis times to be substantially reduced.

Another object of the invention is to propose a method which is completely independent of operator intervention and competence.

Another object of the invention is to propose a method which enables data relative to the metabolic profile to be memorized for later use, and in particular to obtain a data bank relative to the metabolic profile of the patient, usable for retrospective investigations on metabolic changes not specifically related to the initial target.

Another object of the invention is to propose a non-invasive method which is simple to implement, quick to carry out, and economic.

Another object of the invention is to propose an apparatus which receives a biological fluid sample as input and provides as output a diagnostic value indicative of the presence of the colorectal cancer pathological state.

All these objects and others which will be apparent from the ensuing description are attained, according to the invention, by a method with the characteristics indicated in claim 1.

The present invention is further clarified hereinafter by means of a preferred embodiment, with reference to the accompanying drawings, in which:

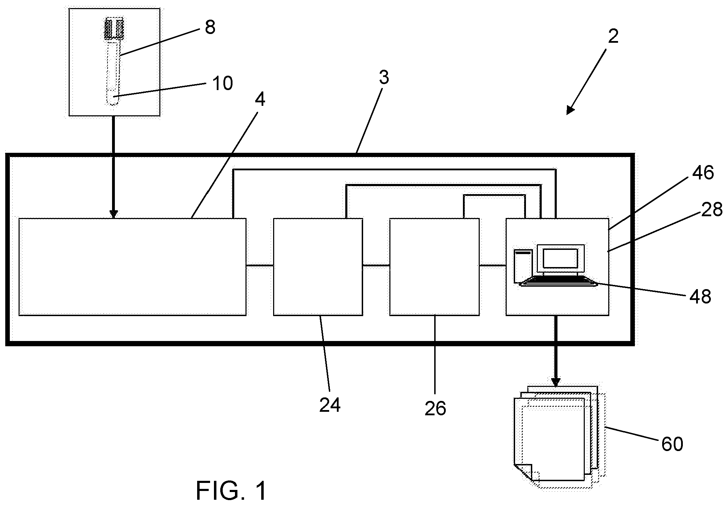

- Figure 1: shows schematically an apparatus according to the invention,

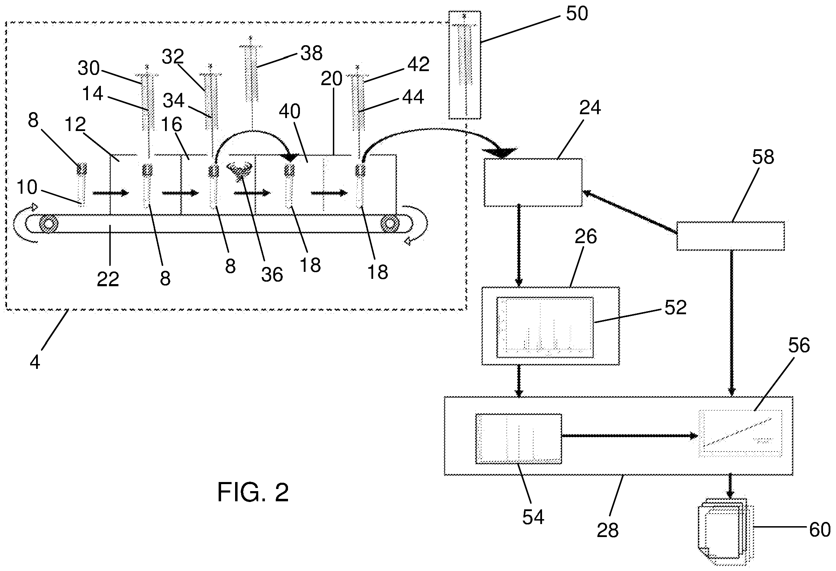

- Figure 2: shows schematically, in greater detail, the steps of the method implemented by the apparatus of Figure 1,
- Figure 3: shows the mass spectra of the fatty acids of interest,
- Figure 4: shows an example of the reconstructed ion chromatograms (RIC) of the fatty acids of interest,
- Figure 5: shows a comparison between two ion chromatograms (RIC) obtained for a subject CRC (i.e. suffering from colorectal cancer) and for a subject "C" (i.e. a healthy control subject),
- Figure 6: shows the concentration distributions of the fatty acids of interest in the four groups analyzed, i.e. the group CRC (comprising subjects with colorectal cancer), the group C (comprising healthy control subjects), the group MAM (comprising subjects with breast cancer), and the group RCU (comprising subjects with ulcerative rectocolitis),
- Figure 7: shows the concentration distributions of the fatty acids of interest in the different stages of advancement of the colorectal cancer, from the pre-cancer form (ADE) to the metastatic CRC IV stage,
- Figure 8: shows the R.O.C curves relative to one of the fatty acids of interest, representing the binary comparison between the subjects of group "C" versus those of group "CRC", versus those of group "MAM" and versus those of group "RCU", and
- Figure 9: shows the R.O.C curves relative to the same fatty acid of interest as Figure 8, representing the binary comparison between the subjects of group "CRC" versus those of group "MAM" and versus those of group "RCU".

As can be seen from the figures, the apparatus 2 for the determination of organic fatty acids, according to the invention, comprises a structure 3 housing an automated system 4 for preparing the sample, intended to receive as input a test tube 8 containing the biological fluid sample 10, specifically the plasma to be analyzed.

In particular, the fatty acids used in the method and in the apparatus 2 according to the invention to determine the presence of the colorectal cancer pathological state comprise at least one of the following saturated fatty acids:
- hexanoic acid (or also caproic) with structure CH₃(CH₂)₄COOH, and identified hereinafter as "C6",
- octanoic acid (or also caprylic) with structure CH₃(CH₂)₆COOH, and identified hereinafter as "C8",
- decanoic acid (or also capric) with structure CH₃(CH₂)₈COOH, and identified hereinafter as "C10",
- dodecanoic acid (or also lauric) with structure CH₃(CH₂)₁₀COOH, and identified hereinafter as "C12".

Preferably, the method and apparatus according to the invention comprises the determination, within the biological fluid sample, of the level of all the aforesaid fatty acids of interest, i.e. C6, C8, C10 and C12.

The automated system 4 for preparing the sample, housed within the structure 3, comprises:
- a first automated station 12 for the addition of a reference standard 14, required to carrying out the measurements within the plasma sample to be analyzed;
- a second automated station 16 for selecting the C6, C8, C10, C12 fatty acids present within the biological fluid sample 10 and their transfer into a second test tube 18;
- a third automated station 20 for the derivatization of the C6, C8, C10, C12 fatty acids,
- robotic means 22 for moving the test tubes 8 and 18 between the various stations; these are preferably traditional movement means.

The following are also housed within the structure 3:
- a gas chromatograph 24 operating under traditional chromatograph conditions, or in accordance with the GCxGC technique or, preferably, in accordance with the fast chromatography technique; in particular, this latter enables a drastic reduction in the analysis time, passing for example from tens of minutes to 5-7 minutes;
- a mass spectrometer 26 positioned at the exit of the gas chromatograph 24; the mass spectrometer 26 is preferably of TOF type, i.e. of "time of flight" type; alternatively, a flame ionization detector (FID) or other mass spectrometry systems (quadrupole, ion trap or other systems) can be provided,
- an electronic processor unit 28 for the data obtained by the mass spectrometer 26 and programmed for determining the level of C6, C8, C10, C12 fatty acids.

In greater detail, the first station 12 is provided with a first automated syringe 30 for adding the reference standard 14 to the interior of the test tube 8.

The second station 16 comprises:
- a second automated syringe 32 for injecting a known volume of extractive mixture 34 into the interior of the test tube 8,
- means 36 for agitating the content of the test tube 8, preferably by ultrasound, to facilitate the extractive process,
- a cooling system using the Peltier effect (not shown),
- a third automated syringe 38 which withdraws the previously separated organic phase from the test tube 8, in order to feed it into the second test tube 18.

The third automated station 20 comprises:
- a zone 40 provided with means (not shown) for heating the solvent contained in the second test tube 18 and evaporating it into a stream of nitrogen,
- a fourth automated syringe 42 for adding a derivatization mixture 44 to the interior of the second test tube 18.

The apparatus 2 also comprises a command and control unit 46 for controlling the individual stations and their components, for their coordination, and for moving between them the test tubes containing the biological fluid sample 10 to be analyzed.

Advantageously, the command and control unit 46 and the electronic processor unit 28 use the same electronic unit 48; in particular, this latter is provided with a traditional interface for dialogue with the operator.

The operation of the apparatus 2 according to the invention is as follows.

The test tube 8 closed at its top by a baffle and containing in its interior the biological fluid sample to be analyzed, preferably of 200 µlitres, is inserted into the housing structure 3 of the apparatus 2 such as to be able to easily and quickly reach the automated system 4 for preparing the sample to be analyzed.

At the automated station 12 of the automated system 4, the reference standard 14 required for carrying out the successive quantitative measurements is then added by the automated syringe 30 to the sample 10 ; in particularly, 10 µlitres of hexane containing the internal reference standard at a concentration of 10 ng/µlitre are added.

The test tube 8 with the biological fluid sample 10 then passes to the station 16 for selecting the C6, C8, C10, C12 fatty acids, by extraction. In this station, the extractive mixture 34 is added by the second automated syringe 32 to the plasma sample 10 contained in the test tube 8, in order to extract the C6, C8, C10, C12 fatty acids from the plasma sample 10. The extractive mixture 34 comprises one or more solvents in a well defined molar ratio based on the C6, C8, C10, C12 fatty acids to be extracted and analyzed; the solvent quantity in the mixture 34 depends on the volume of the biological fluid sample 10 contained in the test tube 8.

For example, the extractive mixture 34 consists of 800 µlitres of chloroform (CHCl₃), 400 µlitres of methanol (MeOH) and 100 µlitres of 0.05M sodium chloride (NaCI) solution. The addition of the extractive mixture 34 to the interior of the test tube 8 results in stratification of the content of this latter, with the organic phase comprising the solvent and the C6, C8, C10, C12 fatty acids defining the lower layer, and the plasmatic layer defining the upper layer.

The organic phase is then withdrawn from the biological fluid sample 10 present in the test tube 8 in station 16 by the third automated syringe 38, and transferred into the second test tube 18. In particular, the insertion of the needle of the third automated syringe 38 into the test tube 8 is suitably controlled on the basis of the quantity of extractive mixture 34 used, such as to enable said needle to reach the bottom portion of the test tube 8 in which the organic phase is stratified.

Moreover, depending on the extractive mixture 34 to be used, the extraction of the organic phase from the test tube 8 can be suitably followed by a step of solvent evaporation. In particular, during this step, the sample contained in the test tube 8 is agitated for some minutes, preferably for 2 minutes, by ultrasound produced by the means 36. The content of the test tube 8 is then allowed to stand for some minutes, for example 5 minutes, to enable and facilitate separation of the organic phase containing the C6, C8, C10, C12 fatty acids from the plasmatic phase. Advantageously, this separation can be accelerated by cooling the test tube 8 by the Peltier effect.

The automated system 4 for preparing the sample also advantageously comprises the automated station 20 for derivatization of the C6, C8, C10, C12 organic acids. In this station, the extracted fatty acids contained in the second test tube 18 are converted into trimethylsilyl derivatives by the silylation process. Essentially, in this manner, a less polar version of the fatty acids is formed, to enable their separation within the gas chromatograph 24.

In particular, in the third station 20, the second test tube 18 containing the previously extracted organic phase, is firstly dried in a stream of nitrogen by the means provided in the zone 40 of the station 20, such as to enable the solvent present in the extracted organic phase to be evaporated. The content of the second test tube 18 is then treated with the derivatization mixture 44 injected by the fourth automated syringe 42. For example, the derivatization mixture 44 comprises 50 µlitres of bis-trifluoro acetamide (BSTFA), 0.1% of trimethylchlorosilane (TMCS) and 50 µlitres of n-hexane. Finally, the whole mixture is heated to about 70°C for substantially 30 minutes.

The sample prepared in this manner is then withdrawn from the second test tube 18 by a fifth automated syringe 50 and injected into the chromatograph 24. In particular, the chromatographic separation can be effected under traditional chromatographic conditions or in accordance with the bi-dimensional gas chromatographic technique (GCxGC) or, preferably, in accordance with the technique known as fast chromatography.

In particular, the chromatography column of the chromatograph 24 has a length of about 10 metres and operates under fast chromatography conditions, i.e. of substantially 0.1 mm of inner diameter, 0.1 mm of stationary phase thickness, and 0.3 ml/min of He flow through the column.

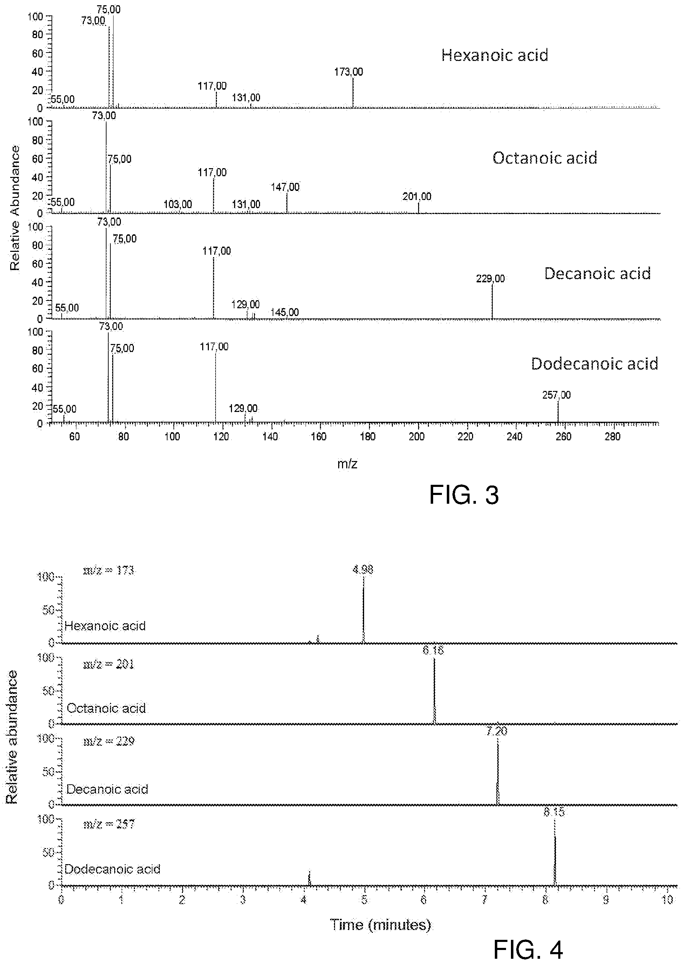

The mass spectrometer 26 is associated with the exit of the chromatograph 24 to carry out the analysis in mass spectrometry. Essentially, this latter subjects the molecular species separated by the gas chromatography to electronic ionization such as to obtain their mass spectrum 52 (see Figure 3).

Preferably, the mass spectrometer 26 used is a "time of flight" (TOF) analyzer in that it enables the chromatographic peaks obtained by fast chromatography conditions to be described with high accuracy.

In greater detail, the spectrometer 26 carries out a series of sequential scans, at a frequency preferably higher than 50 Hz, in such a manner as to obtain a plurality of data, which are then fed to the electronic processor 28 and memorized therein. Advantageously, the data memorized in the processor 28 are filed and organized into a suitable data bank to facilitate their access, selection and processing.

In the processor 28 a programme is loaded which, based on the analysis of the reconstructed ion chromatograms (RIC) 54 (see Figure 4), determines the level of the organic acids having 6, 8, 10 and 12 carbon atom chains (i.e. C6, C8, C10, C12). In particular, these data are obtained by processing the mass spectra 52 memorized in the processor 28 and represent the chromatographic areas of ions characteristic of said fatty acids of interest.

After reconstructing the chromatographic areas of ions of the fatty acids of interest, the programme implemented in the processor 28 carries out a comparison 56, using straight-line calibration or other suitable mathematical/statistical means, with the chromatographic areas of the ions relative to the reference standard 58 memorized in the processor 28.

The programme implemented in the processor 28 then provides as output a series of data 60 which represent the levels of the C6, C8, C10, C12 fatty acids present in the biological fluid sample 10 analyzed, and the results of the comparison between the data 54 and the reference standard 58, to obtain the relative diagnostic information.

In greater detail, to obtain the diagnostic significance of the fatty acid concentration levels obtained in this manner, the processor 28 is configured to compare these levels with the threshold values (cut-offs), which are statistically defined and up-datable, such as to establish whether the determined levels are above or below said threshold values, and hence diagnose the presence or absence of colorectal cancer.

The processor 28 can also be programmed to produce as output a printable report containing the results of the analyses carried out. In particular, the report contains the data of the levels of C6, C8, C10, C12 fatty acids present in the analyzed biological fluid sample 10, and/or the diagnostic significance of said levels.

The processor 28 can also be programmed to determine the relationship between the data representative of the levels of C6, C8, C10, C12 fatty acids present in the analyzed biological fluid sample 10, such as for example the relationship between C10 and C8.

As an alternative to the spectrometer 26, a flame ionization detector (FID) can be associated with the exit of the chromatograph 24. In this case, the quantitative analytical information of interest is suitably based on the retention time.

If a mass spectrometer 26 is used as the detector, the reference standard 14 is a solution comprising the said C6, C8, C10, C12 fatty acids to be analyzed labelled with at least one stable isotope (i.e. containing one or more atoms of ¹³C or ²H); if instead a flame ionization detector (FID) is used, the reference standard 14 is a solution comprising an organic acid with an odd number of C atoms. The use of these reference standards is particularly advantageous as it enables an accurate quantitative analytical results to be obtained.

All the aforedescribed operations carried out by the automated syringes 30, 32, 38, 42 and 50 can in fact be carried out by a single automated syringe or by one or two separate automated syringes.

The biological fluid samples 10 which can be analyzed by the method and apparatus according to the invention comprise plasma, blood, saliva, urine, sinovial fluid and other biological fluids. In particular, in the case in which the biological fluid sample 10 to be analyzed is blood, the sample centrifuging stage can be advantageously avoided, but which instead is essential in the case of plasma.

Using the method and apparatus according to the invention, a study was carried out on 94 plasma samples divided into four groups:
- 21 samples of control subjects (group "C"), to verify the diagnostic specificity of the analyzed fatty acids in correctly identifying healthy subjects,
- 39 samples of subjects with colorectal cancer (group "CRC"), to verify the diagnostic sensitivity of the analyzed fatty acids in correctly identifying unwell subjects,
- 14 samples of subjects with breast cancer (group "MAM"), to verify the diagnostic sensitivity of the analyzed fatty acids in correctly identifying those subjects suffering from CRC compared with subjects suffering from other tumoral forms (organ specificity),
- 20 samples of subjects with ulcerative rectocolitis (group "RCU"), to verify the diagnostic sensitivity of the analyzed fatty acids in correctly identifying those subjects suffering from CRC compared with subjects suffering from colorectal chronic inflammatory pathologies.

Said groups did not differ statistically in terms of age and sex; moreover, two aliquots were extracted for each sample, and both were analyzed.

The levels of C6, C8, C10, C12 fatty acids were then determined for each of these plasma samples using the method and apparatus according to the invention.

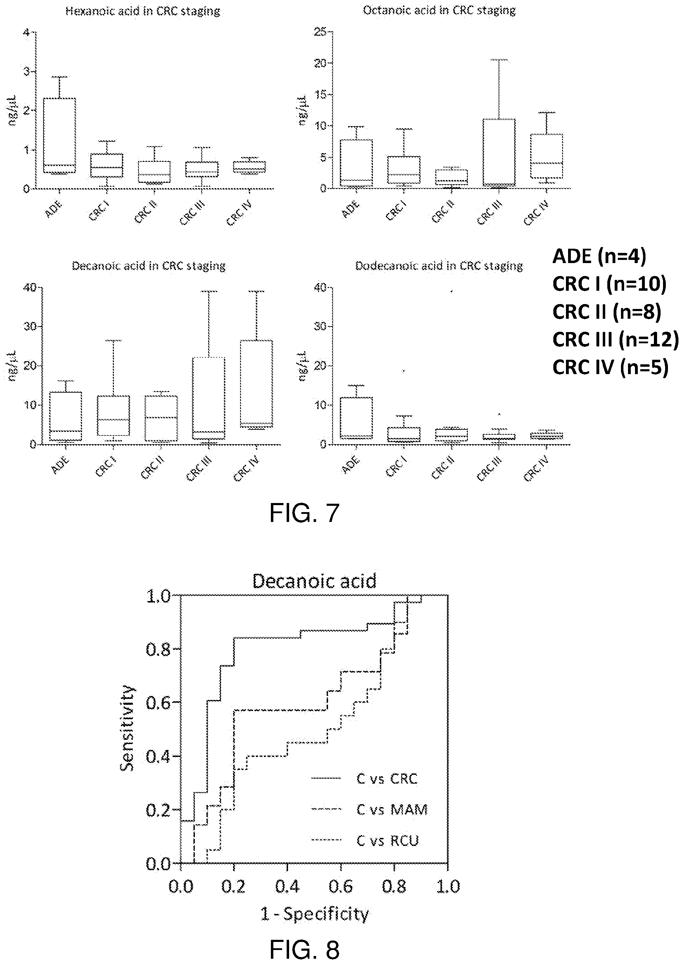

In addition, as shown in Figure 7, for those subjects with colorectal cancer (group "CRC"), the C6, C8, C10, C12 fatty acids levels were correlated with the state of advancement of the pathology ("ADE" for adenoma, "CRC" I for first stage colorectal cancer, "CRC II" for second stage colorectal cancer, "CRC III" for third stage colorectal cancer, "CRC IV" for fourth stage colorectal cancer).

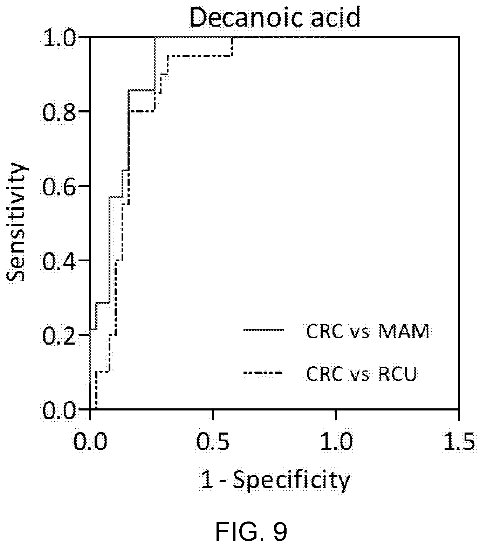

Hence, from the data presented in Figures from 6 to 9, it is apparent that:
- C6 hexanoic acid is present in comparable concentrations in the subjects of control group "C" and in the subjects with ulcerative rectocolitis (group "RCU"), but diminishes significantly in those subjects with colorectal cancer (group "CRC") and with breast cancer (group "MAM"),
- C8 octanoic acid and C10 decanoic acid are present in significantly greater concentration in the samples of subjects with colorectal cancer (group "CRC") than of subjects with ulcerative rectocolitis (group "RCU") and with breast cancer (group "MAM"),
- C10 decanoic acid is present in significantly greater concentration in the samples of subjects with colorectal cancer (group "CRC") than of the control subjects "C",
- C10 decanoic acid already increases in concentration in the pre-cancerous lesions (ADE) and its concentration levels remain virtually constant during the CRC development stages (from CRC 1 to CRC IV, see Figure 7), this making it useful for early diagnosis of CRC development,
- the high C10 decanoic acid levels make it an effective biomarker for diagnosis of colorectal cancer, in that the A.U.C of the R.O.C curves representing the binary comparison subjects "C" versus "CRC" is greater than 0.8 (see Figure 8),
- the high C10 decanoic acid levels make it an effective biomarker specifically for diagnosis of colorectal cancer compared with other pathological states (such as breast cancer or ulcerative rectocolitis), in that the A.U.C of the R.O.C curve representing the binary comparison between the subjects "CRC" versus the subjects "MAM" and versus the subjects "RCU" is in both cases greater than 0.8 (see Figure 9).

In particular, it demonstrates that the organic acid with ten carbon atoms (C10) is particularly interesting for diagnosis of colorectal cancer, this being in line with the fact that this acid is able to bind to a subgroup of the family of nuclear peroxisome proliferator-activated receptors (PPAR), which are involved in the development and modulation of colorectal cancer.

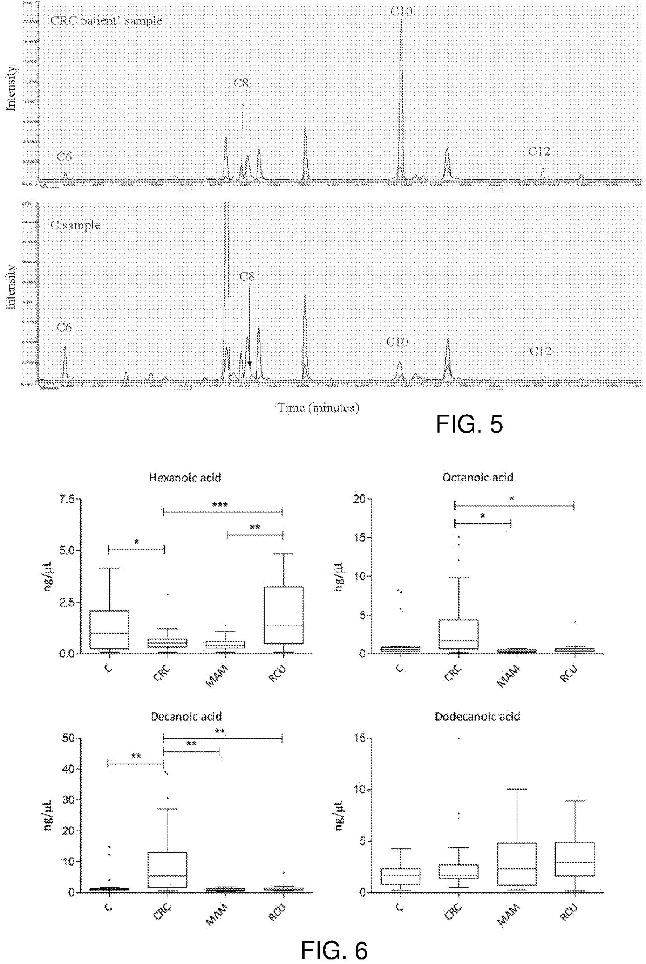

By way of non-limiting example, on the basis of said study, and in particular on the basis of the statistical analysis of the data obtained, as represented in Figure 6, in order to provide a datum useful for diagnostic purposes in terms of C10 decanoic acid found in the plasma, a cut-off value of about 1.3 ng/µlitre was identified. This signifies that if with the method and with the apparatus according to the invention, a C10 decanoic acid level in the plasma substantially greater than about 1.3 ng/µlitre is determined, a datum has been obtained indicative of the presence of colorectal cancer which can be used for diagnosis purposes.

From the aforegoing it is apparent that the method and the apparatus according to the invention is particularly advantage in that:
- they provide in non-invasive manner reliable data for diagnostic purposes, usable at clinical level, of the possible presence of colorectal cancer; in particular, of all possible metabolites, only the C6, C8, C10, C12 fatty acids have been suitably selected specifically as colorectal cancer diagnostic biomarkers,
- they enable analysis times to be substantially reduced, in particularly by virtue of the use of fast chromatography, while at the same time maintaining the same chromatographic resolution; in greater detail, the analysis times are of about 15 minutes against the 50 minutes (45 minutes for analysis plus 5 minutes for reconditioning) required with traditional methods,
- they are completely automated and require no operator intervention; this enables greater operator safety to be achieved plus evaluations independent of operator dexterity and experience,
- the use of extremely small solvent volumes for sample preparation makes the method compatible with the environment in regard to operator health,
- they are simple to implement and use, and are particularly economic,
- they enable data relative to the metabolic profile to be memorized for later use or for different and further evaluation and in-depth study.

## Claims

1. A method for the diagnosis of colorectal cancer by determining the level of fatty acids present in a biological fluid sample (10), comprising the steps of:
- adding a reference standard (14) to the biological fluid sample (10) to be analyzed,
- selecting the fatty acids of interest from the thus additivated biological fluid sample, using an extractive mixture (34) comprising at least one solvent,
- derivatizing said sample using at least one chemical reagent,
- injecting into the gas chromatograph (24) an aliquot of the extracted fatty acids of interest,
- implementing a gas chromatography separation,
- subjecting the molecular species separated by the gas chromatograph (24) to ionization, and analyzing the signals obtained in this manner by a detector,
- processing the data obtained by said detector, to obtain values representative of the level of said fatty acids of interest within said biological fluid sample (10),
and **characterised in that** said fatty acids of interest comprise the decanoic acid.

2. A method as claimed in claim 1, **characterised in that** said fatty acids of interest further comprise, in addition to said decanoic acid, also hexanoic acid, octanoic acid and/or dodecanoic acid.

3. A method as claimed in one or more of the preceding claims, **characterised in that** the gas chromatograph separation is implemented by the fast chromatography method or by the GCxGC method.

4. A method as claimed in one or more of the preceding claims, **characterised in that** said detector is a mass spectrometer or a "time of flight" mass spectrometer (26) or is of flame ionization type (FID).

5. A method as claimed in one or more of the preceding claims, **characterised in that** said reference standard (14) is a solution comprising said fatty acids of interest labelled with at least one stable isotope or is a solution comprising an organic acid with an odd number of C atoms.

6. A method as claimed in one or more of the preceding claims, **characterised by** derivatizing said selected aliquot containing said fatty acids of interest.

7. A method as claimed in one or more of the preceding claims, **characterised by** preceding the derivatization step, by a step of evaporating the solvent present in the component which was previously selected containing the fatty acids of interest of the biological fluid sample (10).

8. A method as claimed in one or more of the preceding claims, **characterised by** comparing the processed data (54) obtained by said detector and representative of the levels of said fatty acids within said biological fluid (10), with the data relative to the reference standards (58), in order to obtain a quantitative measurement which gives a diagnostic significance to said data.

9. Use of an apparatus (2) for diagnosing colorectal cancer by determining the level of fatty acids in a biological fluid sample (10) according the method as claimed in one or more of the preceding claims, said apparatus comprising within a single housing structure (3):
- robotic means (22) for moving at least one test tube (8, 18), containing a sample of biological fluid to be analyzed, between a station (12) in which a reference standard (14) is added and a station (16) for selecting the fatty acids of interest from the additivated biological fluid sample,
- a gas chromatograph (24),
- a detector (26) positioned at the outlet of said gas chromatograph (24),
- a control unit (46) for the entire apparatus,
- a processing unit (28) for the data obtained by said detector

10. Use as claimed in the preceding claim, **characterised in that** said station (16) in which said fatty acids of interest are selected comprises means (32) for injecting a known volume of extractive mixture into said test tube (8) containing the biological fluid sample.

11. Use as claimed in the claims 9 or 10, **characterised in that** said selection station (16) for the fatty acids of interest comprises means (38) for extracting the fatty acids of interest from the biological sample from a first test tube (8) and transferring them into a second test tube (18), means (36) for agitating the content of the test tube (8) containing the biological fluid sample and means (40) for heating and evaporating the solvent present in the biological fluid sample contained within the test tube (18).

12. Use as claimed in one or more of the claims 9-11, **characterised in that** the control unit (46) for the entire apparatus, and the data processing unit (28), are implemented in one and the same electronic unit (48) and **in that** said data processing unit (28) comprises means for memorizing all the data obtained by said detector (26) in suitable data banks.

13. Use as claimed in one or more of the claims 9-12, **characterised in that** within said data processing unit (28), a programme is implemented which, based on the analysis of signals (52) which are reconstructed from the data (54) obtained by the detector (26), determines the level of said fatty acids of interest present within the biological fluid sample (10).

14. Use as claimed in one or more of the claims 9-13, **characterised in that** within said data processing unit (28), a programme is implemented for performing a statistical comparison (56) between the reference standards (58) supplied to the processor (28) and the processed data (54) obtained from the detector (26) and representative of the levels of said fatty acids of interest within said biological fluid sample (10).

15. Use as claimed in one or more of the claims 9-14, **characterised in that** said processing unit (28) is programmed to produce as output a printable report containing data on the levels of said fatty acids of interest present within the biological fluid sample (10) analyzed and/or the diagnostic significance of said levels.

## Patentansprüche

1. Verfahren zur Diagnose von Kolorektalkrebs durch Bestimmung des Fettsäurespiegels in einer biologischen Flüssigkeitsprobe (10), umfassend die Schritte:
- Zugeben eines Referenzstandards (14) zu der zu analysierenden biologischen Flüssigkeitsprobe (10),
- Auswählen der Fettsäuren von Interesse aus der somit additivierten biologischen Flüssigkeitsprobe unter Verwendung einer extraktiven Mischung (34), die mindestens ein Lösungsmittel umfasst,
- Derivatisieren der Probe unter Verwendung von mindestens einem chemischen Reaktionsmittel,
- Injizieren eines Aliquots der extrahierten Fettsäuren von Interesse in den Gaschromatographen (24),
- Durchführen einer Gaschromatographie-Trennung,
- Unterziehen der molekularen Spezies, die durch den Gaschromatographen (24) getrennt wurde, einer Ionisierung und Analysieren der auf diese Weise durch einen Detektor erhaltenen Signale,
- Verarbeiten der vom Detektor erhaltenen Daten, um Werte zu erhalten, die für den Spiegel der Fettsäuren von Interesse in der biologischen Flüssigkeitsprobe (10) repräsentativ sind,
und **dadurch gekennzeichnet, dass** die Fettsäuren von Interesse die Decansäure umfassen.

2. Verfahren nach Anspruch 1, **dadurch gekennzeichnet, dass** die Fettsäuren von Interesse zusätzlich zur Decansäure ferner auch Hexansäure, Octansäure und/oder Dodecansäure umfassen.

3. Verfahren nach einem oder mehreren der vorhergehenden Ansprüche, **dadurch gekennzeichnet, dass** die Gaschromatographie-Trennung durch das Schnellchromatographieverfahren oder durch das GCxGC-Verfahren durchgeführt wird.

4. Verfahren nach einem oder mehreren der vorhergehenden Ansprüche, **dadurch gekennzeichnet, dass** der Detektor ein Massenspektrometer oder ein "Flugzeit"-Massenspektrometer (26) ist oder vom Flammenionisations-Typ (FID) ist.

5. Verfahren nach einem oder mehreren der vorhergehenden Ansprüche, **dadurch gekennzeichnet, dass** der Referenzstandard (14) eine Lösung ist, die die mit mindestens einem stabilen Isotop markierten Fettsäuren von Interesse umfasst, oder eine Lösung ist, die eine organische Säure mit einer ungeraden Anzahl von C-Atomen umfasst.

6. Verfahren nach einem oder mehreren der vorhergehenden Ansprüche, **gekennzeichnet durch** das Derivatisieren des ausgewählten Aliquots, das die Fettsäuren von Interesse enthält.

7. Verfahren nach einem oder mehreren der vorhergehenden Ansprüche, **dadurch gekennzeichnet, dass** dem Derivatisierungsschritt ein Schritt des Verdampfens des Lösungsmittels vorausgeht, das in der Komponente vorhanden ist, die zuvor ausgewählt wurde und die Fettsäuren von Interesse der biologischen Flüssigkeitsprobe (10) enthält.

8. Verfahren nach einem oder mehreren der vorhergehenden Ansprüche, **gekennzeichnet durch** das Vergleichen der verarbeiteten Daten (54), die durch den Detektor erhalten wurden und für die Spiegel der Fettsäuren in der biologischen Flüssigkeit (10) repräsentativ sind, mit den Daten in Bezug auf die Referenzstandards (58), um eine quantitative Messung zu erhalten, die den Daten eine diagnostische Signifikanz verleiht.

9. Verwendung einer Vorrichtung (2) zum Diagnostizieren von Kolorektalkrebs durch Bestimmen des Fettsäurespiegels in einer biologischen Flüssigkeitsprobe (10) gemäß dem Verfahren nach einem oder mehreren der vorhergehenden Ansprüche, wobei die Vorrichtung innerhalb einer einzelnen Gehäusestruktur (3) umfasst:
- Robotermittel (22) zum Bewegen mindestens eines Teströhrchens (8, 18), das eine Probe einer zu analysierenden biologischen Flüssigkeit enthält, zwischen einer Station (12), in der ein Referenzstandard (14) zugegeben wird, und einer Station (16) zur Auswahl der Fettsäuren von Interesse aus der additivierten biologischen Flüssigkeitsprobe,
- einen Gaschromatographen (24),
- einen Detektor (26), der am Ausgang des Gaschromatographen (24) angeordnet ist,
- eine Steuereinheit (46) für die gesamte Vorrichtung,
- eine Verarbeitungseinheit (28) für die von dem Detektor erhaltenen Daten.

10. Verwendung nach dem vorhergehenden Anspruch, **dadurch gekennzeichnet, dass** die Station (16), in der die Fettsäuren von Interesse ausgewählt werden, Mittel (32) zum Einspritzen eines bekannten Volumens einer extraktiven Mischung in das die biologische Flüssigkeitsprobe enthaltende Teströhrchen (8) umfasst.

11. Verwendung nach den Ansprüchen 9 oder 10, **dadurch gekennzeichnet, dass** die Auswahlstation (16) für die Fettsäuren von Interesse Mittel (38) zum Extrahieren der Fettsäuren von Interesse aus der biologischen Probe aus einem ersten Teströhrchen (8) und zum Überführen dieser in ein zweites Teströhrchen (18), Mittel (36) zum Schütteln des Inhalts des Teströhrchens (8), das die biologische Flüssigkeitsprobe enthält, und Mittel (40) zum Erwärmen und Verdampfen des Lösungsmittels umfasst, das in der im Teströhrchen (18) enthaltenen biologischen Flüssigkeitsprobe vorhanden ist.

12. Verwendung nach einem oder mehreren der Ansprüche 9-11, **dadurch gekennzeichnet, dass** die Steuereinheit (46) für die gesamte Vorrichtung und die Datenverarbeitungseinheit (28) in ein und derselben elektronischen Einheit (48) umgesetzt sind, und dass die Datenverarbeitungseinheit (28) Mittel zum Speichern aller von dem Detektor (26) erhaltenen Daten in geeigneten Datenbanken umfasst.

13. Verwendung nach einem oder mehreren der Ansprüche 9-12, **dadurch gekennzeichnet, dass** in der Datenverarbeitungseinheit (28) ein Programm implementiert ist, das auf Basis der Analyse von Signalen (52), die aus den durch den Detektor (26) erhaltenen Daten (54) rekonstruiert werden, den Spiegel der Fettsäuren von Interesse, die in der biologischen Flüssigkeitsprobe (10) vorhanden sind, bestimmt.

14. Verwendung nach einem oder mehreren der Ansprüche 9-13, **dadurch gekennzeichnet, dass** in der Datenverarbeitungseinheit (28) ein Programm implementiert ist, um einen statistischen Vergleich (56) zwischen den Referenzstandards (58), die dem Prozessor (28) geliefert werden, und den verarbeiteten Daten (54), die von dem Detektor (26) erhalten werden und die Spiegel der Fettsäuren von Interesse in der biologischen Flüssigkeitsprobe (10) repräsentieren, durchzuführen.

15. Verwendung nach einem oder mehreren der Ansprüche 9-14, **dadurch gekennzeichnet, dass** die Verarbeitungseinheit (28) programmiert ist, um einen druckbaren Bericht als Ausgabe zu erzeugen, der Daten über die Spiegel der Fettsäuren von Interesse, die in der analysierten biologischen Flüssigkeitsprobe (10) vorhanden sind, und/oder die diagnostische Signifikanz der Spiegel enthält.

## Revendications

1. Une méthode pour diagnostiquer le cancer colorectal par la détermination du taux d'acides gras présents dans un échantillon de fluide biologique (10), comprenant les étapes suivantes:
- l'ajout d'un étalon de référence (14) à l'échantillon de fluide biologique (10) à analyser,
- la sélection des acides gras d'intérêt dans l'échantillon de fluide biologique auquel l'étalon de référence a été ajouté, à l'aide d'un mélange d'extraction (34) comprenant au moins un solvant,
- la dérivation dudit échantillon à l'aide d'au moins un réactif chimique,
- l'injection dans le chromatographe en phase gazeuse (24) d'une aliquote d'extrait d'acides gras d'intérêt,
- la réalisation d'une séparation par chromatographie en phase gazeuse,
- le traitement des espèces moléculaires séparées par chromatographie en phase gazeuse (24) par ionisation, et l'analyse des signaux obtenus de cette manière par un détecteur,
- le traitement des données obtenues par ledit détecteur, afin d'obtenir des valeurs représentatives des taux desdits acides gras d'intérêt dans ledit échantillon de fluide biologique (10),
et **caractérisée en ce que** lesdits acides gras d'intérêt comprennent de l'acide décanoïque.

2. Une méthode selon la revendication 1, **caractérisée en ce que** lesdits acides gras d'intérêt comprennent en outre, en plus dudit acide décanoïque, de l'acide héxanoïque, de l'acide octanoïque et/ou de l'acide dodécanoïque.

3. Une méthode selon l'une ou plusieurs des revendications précédentes, **caractérisée en ce que** la séparation par chromatographie en phase gazeuse est réalisée par une méthode de chromatographie rapide ou par une méthode GCxGC.

4. Une méthode selon l'une ou plusieurs des revendications précédentes, **caractérisée en ce que** ledit détecteur est un spectromètre de masse ou un spectromètre de masse « à temps de vol » (26) ou un détecteur à ionisation de flamme (FID).

5. Une méthode selon l'une ou plusieurs des revendications précédentes, **caractérisée en ce que** ledit étalon de référence (14) est une solution contenant lesdits acides gras d'intérêt marqués avec au moins un isotope stable, ou une solution contenant un acide organique avec un nombre impair d'atomes C.

6. Une méthode selon l'une ou plusieurs des revendications précédentes, **caractérisée par** la dérivation de ladite aliquote sélectionnée contenant lesdits acides gras d'intérêt.

7. Une méthode selon l'une ou plusieurs des revendications précédentes, **caractérisée en ce que** l'étape de dérivation est précédée d'une étape d'évaporation du solvant présent dans le composant précédemment sélectionné contenant les acides gras d'intérêt de l'échantillon de fluide biologique (10).

8. Une méthode selon l'une ou plusieurs des revendications précédentes, **caractérisée par** la comparaison des données traitées (54) obtenues par ledit détecteur et représentant des taux desdits acides gras dans ledit fluide biologique (10), avec les données concernant les étalons de référence (58), afin d'obtenir une mesure quantitative donnant une pertinence diagnostique auxdites données.

9. Utilisation d'un appareil (2) pour diagnostiquer le cancer colorectal en déterminant le taux d'acides gras dans un échantillon de fluide biologique (10) selon une méthode telle que revendiquée dans l'une ou plusieurs des revendications précédentes, ledit appareil comprenant au sein d'une structure d'accueil unique (3):
- des dispositifs robotisés (22) pour déplacer au moins un tube à essai (8, 18), contenant un échantillon de fluide biologique à analyser, entre un poste (12) dans lequel un étalon de référence (14) est ajouté et un poste (16) de sélection des acides gras d'intérêt provenant de l'échantillon de fluide biologique auquel l'étalon de référence a été ajouté,
- un chromatographe en phase gazeuse (24),
- un détecteur (26), placé à la sortie dudit chromatographe en phase gazeuse (24),
- une unité de commande (46) pour l'ensemble de l'appareil,
- une unité de traitement (28) des données obtenues par ledit détecteur

10. Utilisation telle que revendiquée dans la revendication précédente, **caractérisée en ce que** ledit poste (16) dans lequel les acides gras d'intérêt sont sélectionnés comprend des dispositifs (32) conçus pour injecter un volume connu de mélange d'extraction dans ledit tube à essai (8) contenant l'échantillon de fluide biologique.

11. Utilisation telle que revendiquée dans la revendication 9 ou 10, **caractérisée en ce que** ledit poste de sélection (16) des acides gras d'intérêt comprend des dispositifs (38) pour extraire les acides gras d'intérêt de l'échantillon de fluide biologique dans un premier tube à essai (8) et les transférer dans un deuxième tube à essai (18), des dispositifs (36) pour agiter le contenu du tube à essai (8) contenant l'échantillon de fluide biologique et des dispositifs (40) pour chauffer et évaporer le solvant présent dans l'échantillon de fluide biologique contenu dans le tube à essai (18).

12. Utilisation telle que revendiquée dans une ou plusieurs des revendications 9 à 11, **caractérisée en ce que** l'unité de commande (46) de l'ensemble de l'appareil et l'unité de traitement de données (28) sont disposées dans une seule et même unité électronique (48), et que ladite unité de traitement de données (28) comprend des dispositifs pour mémoriser toutes les données obtenues par ledit détecteur (26) dans des banques de données adaptées.

13. Utilisation telle que revendiquée dans une ou plusieurs des revendications 9 à 12, **caractérisée en ce qu'**un programme est mis en application dans ladite unité de traitement de données (28), qui, selon l'analyse des signaux (52) reconstitués à partir des données (54) obtenues par le détecteur (26), détermine le taux d'acides gras d'intérêt présents dans l'échantillon de fluide biologique (10).

14. Utilisation telle que revendiquée dans une ou plusieurs des revendications 9 à 13, **caractérisée en ce qu'**un programme est mis en application dans ladite unité de traitement de données (28) pour réaliser une comparaison statistique (56) entre les étalons de références (58) fournis par le processeur (28) et les données traitées (54) obtenues par le détecteur (26), représentantes des taux desdits acides gras d'intérêt dans ledit échantillon de fluide biologique (10).

15. Utilisation telle que revendiquée dans une ou plusieurs des revendications 9 à 14, **caractérisée en ce que** ladite unité de traitement de données (28) est programmée pour produire un rapport imprimable contenant des données sur les taux d'acides gras d'intérêt présents dans l'échantillon de fluide biologique (10) analysé et/ou la pertinence diagnostique desdits taux.
